# EUROPEAN PATENT APPLICATION

(11) **EP 4 036 362 A1**
(43) Date of publication of application: **03.08.2022**
(21) Application number: 21154766.6
(22) Date of filing: 02.02.2021
(51) Int. Cl.: E06B 3/964, E06B 3/968, E04B 1/58, E04H 3/16, E04B 7/06

(54) **CORNER FIXATING UNIT, STRUCTURAL BUILDING COMPRISING THE CORNER FIXATING UNIT AND METHOD FOR ASSEMBLY OF A STRUCTURAL BUILDING**

(71) Applicant: Kokitech, s.r.o., 619 00 Brno (CZ)
(72) Inventor: Frais, William, 47333 Henån (SE); Meca, Martin, 60200 Brno (CZ)
(74) Representative: AWA Sweden AB

(57) **Abstract**

A corner fixating unit, structural building and method for assembly of the structural building. The corner fixating unit comprising a first half portion extending along a first central axis, and a second half portion extending along a second central axis, wherein the half portions are pivotably fixed to each other to allow pivotation of the first elongated half portion in relation to the second elongated half portion in a plane comprising both the first and second central axes, wherein the half portions each further comprises a tensioning depression, the tensioning depression being adapted to engage a tensioning member.

## Description

### Technical Field of the Invention

The present invention relates to a corner fixating unit, a structural building employing the corner fixating unit, a method for assembly of the structural building and use of structural building as a pool roof or a conservatory structure.

### Background

In the field of fixed constructions different fastening means are known to enable fastening two components made out of a particular material together. Existing solutions include fixating the two components together by use of either a nail or screw, if the material is easily penetrable by a nail or screw, such as e.g. wood. Other solutions for fixating components together wherein the material is hard and not easily penetrable, e.g. when the material is any type of metal, are also known. For example, a hole may be provided in each component to allow fastening of the components via a nut and bolt arrangement. Alternatively, metal components may be welded together or fastened together using riveting.

Using adhesives is yet another example of fastening means and, in some scenarios, an additional fixating bracket is added to allow proper fastening of two components together.

A problem with the existing solutions lies particularly in reliantly fixating two metal components together while still allowing the pieces to easily be separated and repurposed or reused, even for intricately shaped metal components. Metal components that are welded together are difficult to separate without damaging the components. Also, in many intricate fixating scenarios, for example when the metal components are metal columns meeting to form a right-angled corner and the columns are to be fixated at the corner, using a nut and bolt arrangement is impractical. Adhesives exhibit poor fastening strength and adding an additional fastening bracket is not always a viable option out of either practical or design considerations.

### Summary of the Invention

In view of the shortcomings of the existing solutions there is a need for an improved corner fixating unit, a structural building employing the corner fixating unit and method for assembling the structural building. It is an object of the present invention to overcome these shortcomings, and to provide such an improved corner fixating unit, structural building and method for assembling the system.

According to a first aspect of the invention there is provided a corner fixating unit comprising a first elongated half portion extending along a first central axis and a second elongated half portion extending along a second central axis. The first and second elongated half portions are pivotably fixed to each other at a respective inner end portion to allow pivotation of the first elongated half portion in relation to the second elongated half portion in a plane comprising both the first and second central axes. The first and second half portions each further comprises a tensioning depression at a respective outer portion extending along the respective first and second axis, the tensioning depression being adapted to engage a tensioning member.

The invention is at least partially based on the understanding that by providing a corner fixating unit with two half portions which are pivotably connected to each other, and wherein each half portion comprises a depression for engaging a tensioning member the corner fixating unit, may enable facilitated mounting of material profiles together. This invention is applicable regardless of the angle at which the material profiles meet. Moreover, the corner fixating unit offers reliable fastening even for intricate geometries with minimal, or no, components being added externally the material profiles. The corner fixating unit may be adapted to be inserted into two respective hollow structures provided in a respective material profile, each hollow structure receiving at least a portion of each elongated half portion. Accordingly, with the corner fixating unit being provided internally in the material profiles the corner fixating unit may be protected from weather conditions and UV radiation which enhances the lifetime of the corner fixating unit.

The corner fixating unit may be especially suited for use in a pool roof or conservatory structure, such as a greenhouse, terrace structure or balcony structure. The corner fixating unit may be made out of a metal material such as aluminum or iron and the same unit may be suited for fixating material profiles with an angle within a range of angles. Accordingly, manufacturing is facilitated as the same type of corner fixating unit may be used in a wide variety of mounting scenarios. Additionally, the corner fixating unit is reusable and may be removed from one structural installation and used in another structural installation.

In some implementations, each tensioning depression is at least partly tapered such that a depression depth increases from an outer portion of the depression in a direction towards the inner portion of each elongated half portion. With such tapered depressions the corner fixating unit is adapted to move (e.g. by sliding in a direction parallel to the respective central axis) into a predetermined mounting position once engaged by a tensioning member. Precise alignment of the corner fixating unit is thereby automatically arranged during installation.

Additionally or alternatively, the first and second elongated half portions of the corner fixating unit are pivotably coupled to each other via a pivotable pin joint. A rotating pin or hinge joint is an efficient way of realizing the pivoting function of the corner fixating unit.

According to a second aspect of the invention there is provided a structural building comprising the corner fixating member according to the first aspect of the invention, a first material profile comprising a first hollow structure adapted to receive the first elongated half portion of the corner fixating unit, a second material profile comprising a second hollow structure adapted to receive the second elongated half portion of the corner fixating unit, and a first and second tensioning member. Wherein the first and the second material profile each further comprises a tensioning member slot intersecting the respective first and second hollow structure so as to enable the first and second tensioning member to engage the first and second tensioning depression of the corner fixating unit respectively. With a hollow structure and a tensioning member slot provided in each material profile the corner fixating unit may be embedded substantially inside the material profiles in a mounted position. In the mounted position the material profiles are held together by the corner fixating unit which is engaged by the tensioning members at the depressions, via the tensioning member receiving slots. The material profiles may be adapted so as to enable the hollow structure of the material profiles to be in communication in an engaging position, with the corner fixating unit located inside the joint hollow structure formed by the hollow structures of each material profile.

In some implementations, the first and second tensioning member slots are threaded and the first and second tensioning member are threaded so as to enable threaded engagement between the tensioning members and the tensioning member slots. The tensioning member slots may be pre-threaded so as to allow repeatedly and threadedly inserting and removing the tensioning members without introducing much wear to the tensioning member slot. Accordingly, the tensioning members and the material profiles may be mounted, dismounted and reused. Furthermore, by threadedly engaging the tensioning member slots with the tensioning members the tensioning members will be more reliantly held towards a position wherein the tensioning member engages the depression of the corner fixating unit. In some implementations wherein the depressions are tapered, the force at which the material profiles are forced together will be coupled to the threading of the tensioning member into the tensioning member slot. Threading the tensioning deeper into the material profile while engaging the corner fixating unit may tighten the abutment of the first material profile to the second material profile. Additionally, the tensioning member may be a screw and/or have a conical engaging end which is adapted to engage the tensioning depressions. With a screw the threaded insertion of the tensioning member may be controlled with any suitable means for threading a screw, such as a screwdriver or wrench.

In some implementations, each tensioning member slot intersects the respective first and second hollow structure substantially perpendicularly. With such layout of the hollow space inside the material profiles (comprised of the hollow structure for the corner fixating unit and the tensioning member slot) the tensioning members will be guided into perpendicular engagement with the depressions of the corner fixating unit by the tensioning member slot. This brings more reliable fixating of the corner fixating unit in the hollow structure inside the material profiles as there is little risk of the depressions of the corner fixating unit sliding out of engagement with the tensioning member, even when put under strain.

In some implementations the structural building further comprises a corner stabilizing member. The corner stabilizing member is fixated to the first material profile and the second material profile further comprises a slot for receiving a portion of the corner stabilizing member fixated to the first material profile or the first and second material profile each comprises a slot for receiving a respective portion of the corner stabilizing member. The corner stabilizing member may thus be an insert which is located inside the corresponding slots of each material profile in the mounted position. The stabilizing member makes the connection between the material profiles more stable and avoid e.g. bending, torsion and compression of the material profiles together in the mounted position. While the corner fixating unit prohibits the material profiles from being pulled apart. In some implementations, to decrease the material cost and weight of the structural building the material profiles feature additional hollow segments to maintain the outer dimensions of the profile but decrease the amount of needed material. In such implementations, the corner stabilizing member ensures that extra material which provides additional structural stability is provided where most needed, i.e. in the corners and where two material profiles are to be mounted together, while much of the material profiles may remain hollow, without inserted elements. The corner stabilizing members is adapted to extend at least partly inside the slot of at least one of the two material profiles. Wherein the slot is a hollow structure which extends through the entire or at least a portion of the material profile.

As mentioned in the above, the corner stabilizing member may additionally or alternatively be a separate component from the material profiles, being inserted into both material profiles during mounting. Alternatively, the corner stabilizing member may be fixated to one material profile, extending outwards from an abutting surface of the material profile, while the other material profile comprises a corresponding slot of receiving the stabilizing corner member. In some embodiments, each of the material profiles comprises a slot for receiving the corner stabilizing member of the other material profile and an extending corner stabilizing member to be received in the other material profile.

In some implementations, the angle between the first and second central axis of the corner fixating member is within a range of 10-170 degrees, preferably within a range of 70-110 degrees when in the mounted position together with the material profiles. Accordingly, the structural building may comprise material profiles meeting at a wide variety of angles, with a same corner fixating unit enabling mounting of all material profiles together.

In some implementations, the material profiles are elongated structural columns. For example, the structural columns or beams make up the main and supporting structure of the structural building. The elongated structural columns may be sized and adapted to be used in to form a pool roof or a conservatory structure. Alternatively or additionally, the material profiles are made out of aluminum.

Thus, the material profiles may serve as structural beams or columns of the structural building and may comprise further means for engaging e.g. drywall segments, plastic screens, glass screens and seal fittings so as to allow construction of a complete structural building. A benefit of this is that the structural building may be easily built from two or more material profiles on site. In most prior solutions, structural buildings such as pool roofs or conservatory structures in metal necessitate delivery of complete prefabricated sections of the structure to the construction site, which makes for cumbersome and inefficient transportation. This is brought by the fact that the most intricate parts of the structure, comprising corners or columns meeting at an angle, are made as one piece or sections of welded together pieces. With the invention, structures such as pool roofs or conservatory structures may be packed with high spatial efficiency as individual columns and be mounted together on site using the corner fixating units and the tensioning members described in the above. Experiments have shown that the time for installing a structural building is decreased by using the corner fixating unit and material profiles of the invention while also the final structural building is structurally more stable in comparison to previous solutions.

According to a third aspect of the invention there is provided a structural building according to the second aspect, used as a pool roof or a conservatory structure. The enhanced stability and modularity of the corner structural building makes it especially well-suited for use as a pool roof, e.g. with sliding segments, or a conservatory structure. During assembly of such a structural building heavy and bulky prefabricated parts may be avoided in preference of smaller parts which facilitates transport efficiency and decreases transport costs. Moreover, no special tools are required for assembly of the parts, e.g. the tensioning members may be provided with hex key or Allen key sockets such that the structural building may be assembled using only a hex key or Allen key to tighten the tensioning members. For example, the customer or intended owner of the structural building may assemble it on his/her own. After use, the parts making up the structural building is easily deconstructed to e.g. be repurposed as a new structural building.

According to a fourth aspect of the invention there is provided a method for assembling a building structure by fixating a first material profile to a second material profile. The method comprising the steps of providing a corner fixating unit according to the first aspect of the invention described in the above; providing first and second tensioning member; inserting the first elongated half portion of the corner fixating unit into a first hollow structure of the first material profile; inserting the second elongated half portion of the corner fixating unit into a second hollow structure of the second material profile; inserting a first and second tensioning member into a respective tensioning member slot of each material profile, wherein each tensioning member slot intersecting the respective first and second hollow structure; and engaging the first and second tensioning depressions of the corner fixating unit with the first and second tensioning members respectively.

The assembly method allows the corner fixating unit to be inserted into the material profiles prior to bringing the material profiles into contact. Even prior to inserting the tensioning members so as to engage the depression of the corner fixating unit the corner fixating unit will enable some structural stability allowing the material profiles to be preliminarily mounted together before being more reliably fixated with the tensioning members. Taking the structural building apart is easily performed by performing the reverse operation. That is, removing the tensioning members and subsequently removing the corner fixating unit from each of the material profiles.

In some implementations the tensioning depressions are tapered and the tensioning member slots comprise threads. The method for assembly may then further comprise the steps of rotating the first tensioning member in the first threaded tensioning member slot so as to engage the tapered tensioning depression of the first elongate half portion of the corner fixating unit, and rotating the second tensioning member in the second threaded tensioning member slot so as to engage the tapered tensioning depression of the second elongate half portion of the corner fixating unit, so that the first material profile is tightened to the second material profile by the engagement of the tensioning members and the tapered tensioning depressions.

When rotating the threaded tensioning members into the respective threaded tensioning member slots an engaging end of the tensioning member is forced towards the respective depression of the corner fixating unit. When the engaging end of the tensioning members are engaging the tapered depressions of the corner fixating unit, the corner fixating unit will be placed under a tension force, counteracted by a compression force acting on the material profiles. Rotating the tensioning members into the tensioning members slots will tighten the material profiles, the corner fixating unit and the tensioning members together. Accordingly, the stability of a structural building is dependent on the threading depth of the tensioning members. To facilitate assembly, the tensioning members may initially be threaded to first position, so as to provide a less stable structural building during construction, to allow some flexibility when installing potential further components of the structural building and subsequently threaded to a second position, which provides a more stable, tighter coupling between the first and second material profiles.

The invention according to the third and fourth aspects features the same or equivalent embodiments and benefits as the invention according to the first and second aspect. Further, any functions described in relation to a method, may have corresponding structural features in a system or unit. It is noted that the invention relates to all possible combinations of features recited in the claims.

### Brief Description of the Drawings

These and other aspects of the present invention will now be described in more detail, with reference to the appended drawings showing exemplar embodiments of the present invention, wherein:
Fig. 1a and 1b illustrates a first and second half portion of a corner fixating unit according to embodiments of the invention.
Fig. 1c and 1d illustrates a corner fixating unit arranged with different angles between the respective central axis according to some embodiments of the present invention.
Fig. 2 depicts a perspective view of a material profile according to some embodiments of the invention with the hollow structures illustrated.
Fig. 3a depicts a side view of a material profile according to some implementations.
Fig. 3b depicts a corner fixating unit inserted into the hollow structure of a material profile.
Fig. 4a illustrates an abutting face of a material profile according to embodiments of the invention.
Fig. 4b illustrates an alternative corner fixating unit.
Fig. 5a illustrates two material profiles and a corner fixating unit according to embodiments of the invention.
Fig. 5b illustrates two material profiles in a mounted position, held together by the corner fixating unit according to some embodiments of the invention.
Fig. 5c illustrates a corner stabilizing member and two material profiles according embodiments of the invention.
Fig. 6 is a flowchart describing a method according some embodiments of the present invention.

### Detailed Description

In the following detailed description, some embodiments of the present invention will be described. However, it is to be understood that features of the different embodiments are exchangeable between the embodiments and may be combined in different ways, unless anything else is specifically indicated. Even though in the following description, numerous details are set forth to provide a more thorough understanding of the present invention, it will be apparent to one skilled in the art that the present invention may be practiced without these details. In other instances, well known constructions or functions are not described in detail, so as not to obscure the present invention.

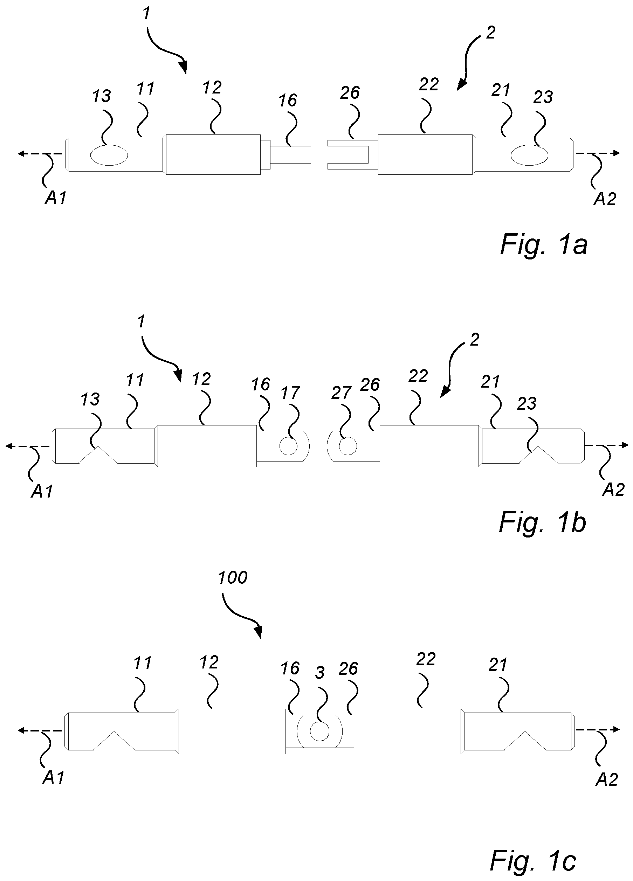

Fig. 1a and 1b depicts portions of the corner fixating unit according to some implementations. The corner fixating unit comprises two half portions 1, 2. The half portions 1, 2 may be elongated and extend along a respective central axis A1, A2 and are adapted to be pivotably attached to each other at a respective inner end portion 12, 22. The half portions 1, 2 may have any suitable shape e.g. be substantially cuboid or cylinder shaped. In some implementations, at least one of the half portions 1, 2 is curved so as to allow insertion into a matching curved hollow structure.

Each half portion 1, 2 further comprises at a respective outer portion 11, 21 a tensioning depression 13, 23 in the respective outer portion 11, 21. The tensioning depressions 13, 23 are adapted to receive a tensioning member so as to reliantly fixate each half portion 1, 2. That is, fixate in the sense that each half portion 1, 2 is prohibited from moving along at the respective central axis A1, A2 pointing from the inner end portion 12, 22 towards the outer portion 11, 21. Further depicted in Fig. 1a and Fig. 1b is exemplary means 16, 26 comprised in the inner end portions 11, 12 for allowing a pivotable coupling of the first half portion 1 to the second half portion 2. In the example shown, the means 16, 26 form a hinge mechanism when the two half portions 1, 2 are brought together. The hinge mechanism allowing a hinge pin to be inserted through holes 17, 27 provided in the means 16, 26 so as to pivotably couple the first and second half portions 1, 2 together and form a corner fixating unit according to some embodiments of the invention.

Further illustrated in Fig. 1a and Fig. 1b are the tensioning depressions 13, 23 of each outer portion 11, 21. The shape of the tensioning depressions 13, 23 may be any shape so long as the depressions 13, 23 generate an indentation in the geometry of each outer portions 11, 21, extending along the respective central axis A1, A2. For example, the tensioning depressions may be conically and/or concavely shaped. A tensioning member inserted into a depression 13, 23 prohibits the half portions from moving in the direction of the respective central axis. In some implementations the depressions 13, 23 are tapered so as to feature a deeper indentation closer to the inner end portions 12, 22 and a shallower indentation closer to the outer portion 11, 21, forming an outer flank.

Additionally, besides this shape of the outer flank of the depressions 13, 23 some implementations further comprise a tapering of the inner flank where the depressions are tapered so as to feature a shallower indentation closer to the inner end portions 12, 22 and deeper indentation closer to the outer portion 11, 21. The outer flank being displaced in the direction of the respective central axis A1, A2 from the inner flank. In these implementations the depressions 13, 23 are adapted to receive a tensioning member such that the respective half portion 1, 2 is forced towards a predetermined fixating position. The predetermined fixating position being dictated by the intersection of the inner and outer depression flanks. The triangular depressions 13, 23 depicted in Fig. 1a and Fig. 1b are merely exemplary, the shape of the depressions may be smooth or appropriately curve-shaped. Moreover, a corner fixating unit may comprise two or more depressions in each half portion 1, 2 distributed along the respective half portion 1, 2 or oriented to face different directions so as to allow engagement of tensioning members in material profiles with differently located tensioning member slots.

The placement and shape of the depressions 13, 23 depicted are merely exemplary. The depression 13 of the first half portion 1 may be placed, oriented or shaped differently and independently from the depression 23 of the second half portion 2. The half portions 1, 2 may have different lengths and dimensions and are to be interpreted as 'half' portions being one out of two portions. Preferably, the design of the corner fixating unit is adapted such that the depressions 13, 23 in the mounted position face directions which facilitates simple insertion of the tensioning members during installation. For example, the placement of the tensioning member slots of the material profiles and the depressions 13, 23 of the corner fixating are adapted with such that all tensioning members are inserted from the inside of the structural building (e.g. to avoid moisture and dirt from the outside getting into the tensioning members slots) or inserted at position easily accessible during construction.

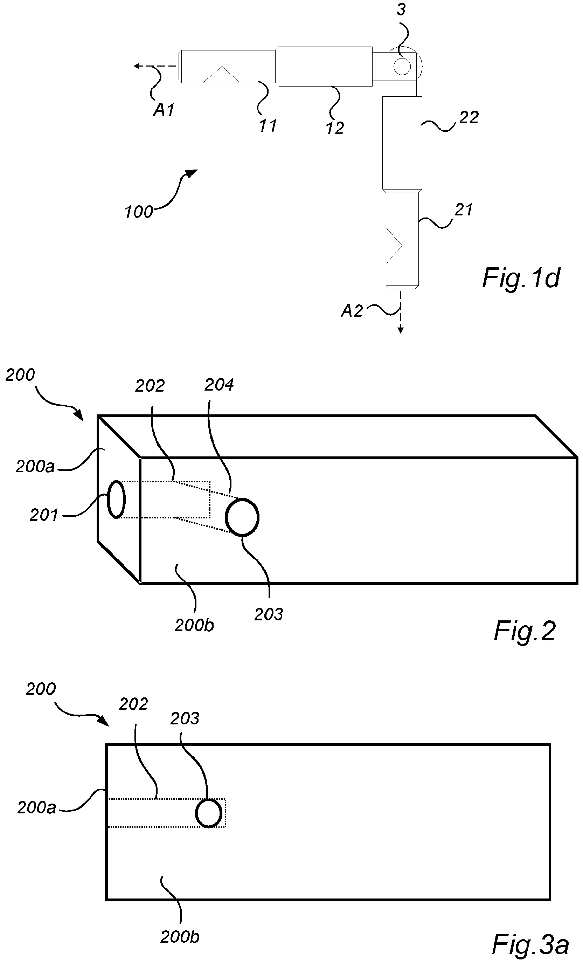

Fig. 1c depicts a corner fixating unit 100 wherein the first and second elongated half portions 1, 2 are pivotably fixated to each other at a pivoting or rotating point 3. The pivoting point 3 may be enabled by a pivoting pin 3 inserted in through holes provided in each of the half portions 1, 2. The corner fixating unit 100 depicted in Fig. 1c is arranged with an angle between the first and second central axis A1, A2 of approximately 180 degrees. With further reference to Fig. 1d there is depicted the corner fixating unit 100 arranged with an angle between the first and second central axis A1, A2 of approximately 90 degrees. Moreover, it is clear that the corner fixating unit may be arranged with any angle between the first and second central axis A1, A2 within a range of approximately 180 degrees and approximately 0 degrees. Some implementations are enabled to be arranged with an angle between the first and second central axis A1, A2 which falls within the range of 180 degrees to 10 degrees, or 170 degrees to 10 degrees. The first and second central axis A1, A2 are defined with respect to the elongation of each half portion 1, 2. Each axis A1, A2 features an inherent directionality, the directionality pointing from the inner end portions 12, 22 (or alternatively from the pivoting point 3) towards the outer portion 11, 21.

Fig. 2 illustrates a perspective view of a material profile 200 comprising a first hollow structure 202 extending into the material profile 200. The material profile comprises an abutting surface 200a in which the opening 201 of the hollow structure 202 is located. The hollow structure 202 is adapted to receive at least a part of a half portion of the corner fixating unit through the hollow structure opening 201. The hollow structure 202 is adapted to receive a half portion of the corner fixating unit such that the tensioning depression of the received half portion is located within the hollow structure 202. That is, the depression of the received half portion is to be placed inside the opening of the hollow structure 202, e.g. so as to align with the tensioning member slot 203, 204.

The material profile 200 further comprises a tensioning member slot 203, 204 which, similar to the hollow structure opening 201 and hollow structure 202, comprises a tensioning member hollow structure 204 and a tensioning member receiving opening 203. The tensioning member slot 203, 204 intersects the hollow structure 202. That is, the tensioning member hollow structure 202 and the hollow structure 202 defines a joint hollow volume 202, 204 inside the material profile 200, wherein the joint hollow volume 202, 204 comprises two separate openings, the tensioning member receiving opening 203 and the abutting face opening 201. In some implementations the hollow structures 202, 204 are elongated so as to enable receiving of an elongated half portion of a corner fixating unit and an elongated tensioning member respectively. In some implementations the hollow structures 202, 204 intersect at substantially right angles. For example, the material profile 200 may be shaped as a cuboid, with the hollow structure 202 extending into the material profile from the hollow structure profile opening 201 provided in the abutting face 200a, towards an opposite far surface. While the tensioning member hollow structure 204 extends into the material profile 200 from the tensioning member receiving opening 201 provided in a side face 200b towards an opposite side surface. The abutting face 200a and the side face being 200b being perpendicular to each other.

Other shapes of the material profile 200 are also possible, wherein the hollow structure 202 and the tensioning member hollow structure 204 intersect at other angles, e.g. between 10 degrees and 170 degrees, such as 30, 45, 60, 75 or 105 degrees. The hollow structures in the material profile may be drilled or be formed by other means in the material profile. Alternatively, the hollow structures are formed during extrusions or casting of the material profiles.

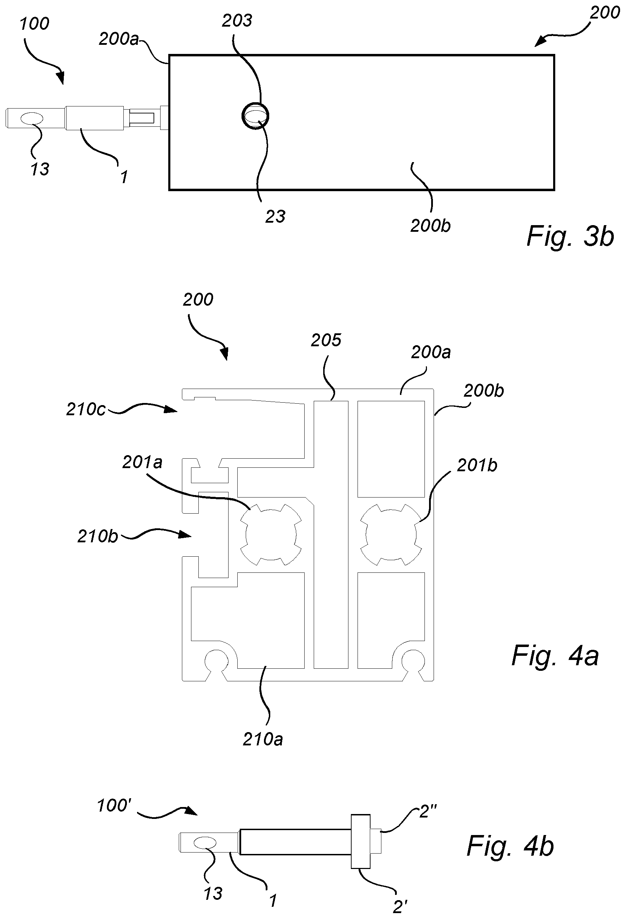

Fig. 3a illustrates a side view of a material profile 200. Viewed from the side in which the tensioning member receiving opening 203 is provided in the side face 200b. The hollow structure 202 adapted to receive a half portion of a corner fixating unit extends into the material profile 200 from the abutting face 200a. With further reference to Fig. 3b the hollow structure 200 has received a corner fixating unit 100 in the hollow structure 202. The second half portion of the corner fixating unit 100 comprising the tensioning depression 23 is substantially inside the material profile 200 and a tensioning member may be inserted into the tensioning depression opening 203 to engage the tensioning depression 23 of the second half portion of the corner fixating unit 100. Which may prohibit the corner fixating unit 100 from being removed from the material profile 200.

The first half portion 1 of the corner fixating unit 100 may also be inserted into a second material profile in a manner similar to the way the second half portion is inserted into the material profile 200. In some implementations, the material profiles are brought into contact such that the abutting face 200a of the material profile abuts a corresponding abutting face of the second material profile. By inserting a tensioning member to engage the depressions 13, 23 of each half portion 1 the two material profiles will be mounted together by the corner fixating unit 100. The pivoting function of the corner fixating unit 100 allows the material profiles 200 to extend in different directions, while still being tightened together by the corner fixating unit 100.

Fig. 4a is a side view of a material profile according to some implementations, observed from the abutting face 200a. In the abutting face 200a there may be one or more openings 201a, 201b provided, each opening 201a, 201b being in communication with a respective hollow structure adapted to receive a half portion of a corner fixating unit. Accordingly, a respective tensioning member receiving opening and tensioning member hollow structure is provided in the side face 200b to allow a respective tensioning member to interact with the depressions of each half portion of each corner fixating unit. The profile of the openings 201a, 201b may be smooth or provided with notches around the perimeter. For example, the shape of the corner fixating unit and the holes 201a, 201b may be adapted such that the tensioning depressions of the corner fixating unit is rotationally aligned with the tensioning member receiving opening of the material profile 200 to further facilitate fastening. That is, the corner fixating unit may have a protrusion which is received in a notch of the opening 201a, 201b such that the tensioning depression will face the direction of the tensioning member receiving opening.

The number of openings 201a, 201b and associated hollow structures provided in the abutting face 200a of a material profile may further be any number greater than one, accordingly the half portion of one or more corner fixating units may be received in a material profile and used simultaneously. Each hollow structure adapted to receive a portion of a corner fixating unit is in communication with a tensioning member receiving slot according to embodiments of the invention. With additional hollow structures adapted to receive corner fixating units provided in each material profile, and additional tensioning members used to engage the depressions of the corner fixating units, the strength at which one material profile may be coupled to another material profile is increased. The opening 201a, 201b may be shaped so as to form a tight fit with an inserted half portion of the corner fixating unit. Moreover, the opening 201a, 201b and a portion of the hollow structure being adjacent to the opening 201a, 201b may be shaped so as to enable the corner fixating unit to be pivoted, while being inside the material profile 200 in the mounted state. Some pivoting arrangements may feature portions that projects outwards once the pivoting angle exceeds a certain angle, to allow use of such pivoting arrangements the openings 201a, 201b and the opening adjacent hollow structure may be shaped accordingly.

Additionally, the abutting face 200a may further be provided with a slot 205 for receiving a corner stabilizing member. The slot 205 may be a slot going through the entire material profile 200 or the slot 205 may be provided only partially through the material profile 200. The corner stabilizing member may be plate shaped and feature a 90-degree L-profile or an L-profile wherein the two extending portions are arranged with respect to each other at a different angle, so as to stabilize the two material profiles in the mounted position. Additionally, some implementations of the material profiles 200 feature structural hollow features 210a which e.g. makes the material profile hollow and enables a lower material profile weight. Alternatively or additionally, the material profiled 200 may feature further mounting structures 210b and 210c which allows the material profile 200 to engage other material profiles or components so as to add further flexibility when constructing structures. For example, a further mounting structure 210b may be a mounting rail extending along the material profile 200. The mounting rail 210b being adapted to receive a sliding member, the sliding member being coupled to a fixating portion, the fixating portion may be a half portion of the corner fixating unit according to embodiments of the invention. Thus, the abutting face of a material profile may be mounted towards a side face of a material profile using the mounting rail 210b and a mounting component comprising a half portion of a corner fixating unit and a sliding member.

With further reference to Fig. 4b an exemplary corner fixating 100' unit with the one half portion replaced with a sliding member 2' is depicted. The sliding member 2' is inserted to the mounting rail 210b and the half portion 1 is inserted into a hollow structure provided in an abutting face of a material profile. By engaging the depression 13 of the half portion 1 with a tensioning member, the abutting face of the material profile is tightened towards the side face (which comprises the mounting rail 210b) of the second material profile. The mounting rail 210b may be shaped as a T-groove which extends along the length of the material profile. The sliding member 2' of the corner fixating unit 100' may further comprise an protrusion 2" configured to support a material profile with a mounting rail 210b, wherein the cross-sectional shape of the mounting rail is dimension to receive the sliding member 2' but not the sliding member 2' with the protrusion 2". Accordingly, tightening a corner fixating unit 100' with a protrusion 2" with a fixating member engaging the depression of the half portion 1 in a first material profile wherein the sliding member 2' is at least partially inserted into the mounting rail 210b of a second material profile and wherein the protrusion 2" is in contact with the abutting face of the second material profile fixates the second material profile towards the abutting face of the first material profile while the protrusion 2" supports the second material profile (e.g. in an upright position). A corner fixating unit 100' facilitates the assembly of structural buildings as fixating units 100' with sliding members 2' comprising a protrusion 2" may support material profiles during assembly and prohibit material profiles from sliding past a predetermined mounting position. The structural building may thus be designed with fixating units 100' with protrusions 2" arranged such that the structural building is easier and more intuitive to assemble.

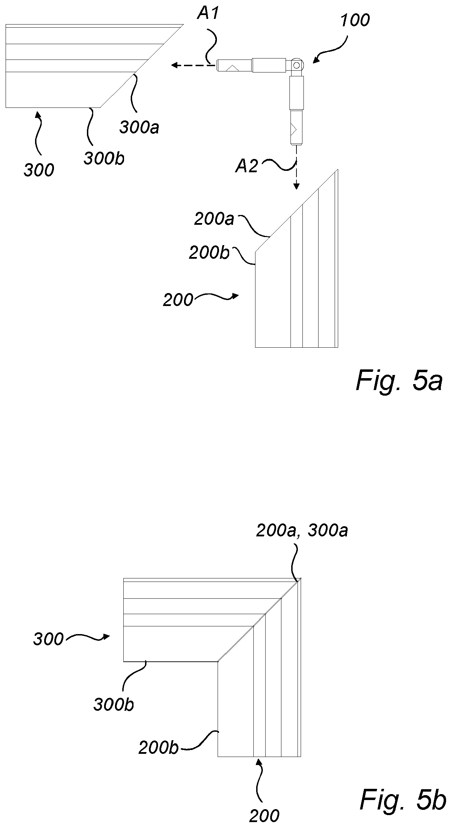

Fig. 5a illustrates a first material profile 200, a second material profile 300 and a corner fixating unit 100. The material profiles 200, 300 each comprise an abutting face 200a, 300a angled at approximately 45 degrees so as to, in the mounted position, form an approximately 90-degree corner angle. The material profiles 200, 300 may e.g. form a ceiling beam 300 meeting a wall column 200.

The corner fixating unit 100 is inserted into the hollow structure provided in the abutting face 200a, 300a of each material profile 200, 300 along the first and second central axis A1, A2 and tensioning members are inserted into the tensioning member slots provided in each material profile. For example, the tensioning member receiving slots are arranged in the respective side faces 200b, 300b of the material profiles 200, 300. With further reference to Fig. 5b, there is depicted the material profiles 200, 300 in the mounted position with the corner fixating 100 member substantially or entirely enclosed by hollow structures of each material profile 200, 300. The abutting face 200a, 300a of each material profile 200, 300 are in abutment. In some implementations the tensioning members are engaging the depressions of the corner fixating unit so as to, via the pivoting point of the corner fixating unit 100 force the abutting faces 200a, 300a with a force. It is understood that with material profiles 200, 300 featuring abutting faces 200a, 300a oriented differently with respect to the extending direction of the material profiles 200, 300 any corner angle, e.g. within a range of 10 degrees to 170 degrees, is achievable. The corner fixating unit 100, being pivotable, is suited for fixating the material profiles 200, 300 for any angle in such a range.

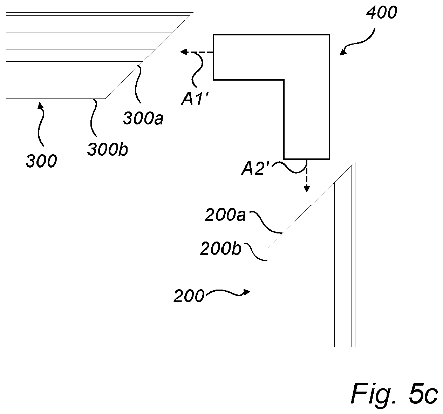

With reference to Fig. 5c there is illustrated the material profiles 200, 300 and the corner stabilizing member 300. The corner stabilizing member 300 may be plate shaped and/or an L-profile. The corner stabilizing member has two half portions extending along respective central axes A1' and A2' so as to form an angle between the central axis A1', A2' suitable for the particular corner to be stabilized. The exemplary corner stabilizing member 400 in Fig. 5c is an L-profile with a 90 degree angle adapted to be inserted into corresponding slots provided in the abutting faces 200a, 300a of the material profiles 200, 300. The corner stabilizing member 400 will add additional support to the material profiles 200, 300 in the mounted position. With further reference to the exemplary orientation of the hollow structures and slot 205 in the abutting face 200a depicted in Fig. 4a it is illustrated how a corner fixating unit and a corner stabilizing member 400 may both be inserted into a material profile 200, 300 so as to complement each other. That is, the slot 205 for the corner stabilizing member 400 is displaced from the opening 201a, 201b adapted to receive the corner fixating unit. Other shapes of the corner stabilizing member 400 is possible, for example the corner stabilizing member 400 may be a rigid rod with two rigid half portions meeting at an angle suited for being inserted in the material profiles 200, 300 and being located inside the material profiles 200, 300 in the mounted position.

In some implementations, one out of the two material profiles 200, 300 to be fixated together in the mounted position comprises a slot 205 while the other material profile 200, 300 comprises a corner stabilizing member fixated so as to extend from the abutting face 200a, 300a. In the mounted position the corner stabilizing member fixated to the material profile is inserted into the slot 205 provided in the abutting face 200a, 300a of the other material profile 200, 300.

To facilitate insertion of the corner stabilizing member 400 the corner stabilizing member 400 may be dimensioned so as to feature some play with respect to the slot 205 into which it is to be inserted during mounting. That is, at least feature some play with respect to the slot during a portion of the insertion process.

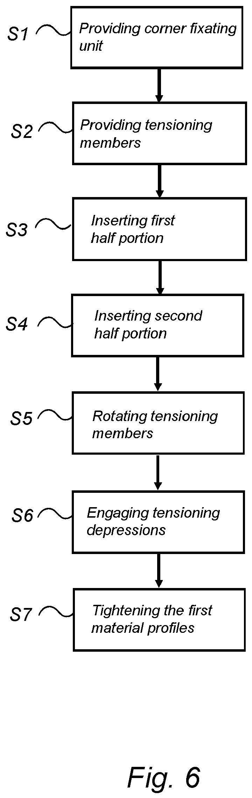

Fig. 6 depicts a flowchart describing a method according to some implementations. At S1 a corner fixating unit according embodiments of the invention is provided. The two half portions of the corner fixating unit are pivotable around a pivoting point so that the angle between the central axes of each half portion is adjustable. At S2 tensioning members are provided, the tensioning members being adapted to be inserted into tensioning slots of the material profiles and engage the depressions of the corner fixating unit. In some implementations the tensioning members are bolts or screws which engage threads provided in the tensioning member slots of the material profiles. With a corner fixating unit provided, at least two tensioning members provided and two material profiles according to embodiments of the invention provided the method may go to step S3 and S4. At S3 and S4 the first and second half portions of the corner fixating unit are inserted into a hollow structure of a respective material profile. S3 and S4 may be performed in any order, or simultaneously.

Subsequently, the method may go to optional step S5 comprising rotating a threaded tensioning member into to a threaded tensioning member slot so as to, at step S6, engage the depression of the corner fixating unit with the tensioning member. By engaging the depression of each half portion of the corner fixating unit with tensioning members at S6 the corner fixating unit will be locked in place with the material profiles in the mounted position. As an optional final step, S7, the tensioning members are rotated into the tensioning member slots and engage the depressions so as to tighten the material profiles against each other. According to some embodiments the depressions may be tapered or conically shaped so as to force the half portion of the corner fixating unit in the direction of the central axis of that half portion. As both tensioning members are brought in engagement with the depressions a force pulling the half portions of the corner fixating unit apart is counteracted by a force tightening the material profiles against each other at the respective abutting faces. The method may comprise repeatedly providing material profiles, tensioning members, corner fixating units and mounting the material profiles together so as to assemble a complete pool roof or conservatory structure. Furthermore, the method may comprise the steps of dismounting the structural building (such as a pool roof or a conservatory structure) and reusing the material profiles, tensioning members and corner fixating unit to construct a new, different or identical, structural building. In some implementations, wherein at least one of the material profiles comprises a mounting rail or slot of for receiving a corner stabilizing member the method comprises at least one of the steps of fixating a material profile side face to an abutting face of another material profile using the mounting rail and fixating unit (comprising an elongated half portion and a sliding member) and inserting the corner stabilizing member into at least one material profile so as so stabilize the corner in the mounted position.

Moreover, the method may comprise providing material profiles with a mounting rail and providing a second type of fixating unit comprising a sliding member according to embodiments of the present invention. The sliding member of the second type of fixating unit being inserted into the mounting rail of a first material profile and a second/opposite portion of the second type of fixating unit being inserted into a hollow structure of another material profile. The second/opposite portion of the second type of fixating unit being provided with a tensioning depression adapted to receive a tensioning member so as to tighten an abutting face of the another material profile against the mounting rail side of the material profile. The second/opposite material profile may serve as a crossbeam witch is in turn fixated to a mounting rail of an additional material profile in the other end. The steps may be repeated so as to construct the entire structural building.

The skilled person in the art realizes that the present invention by no means is limited to the embodiments described above. The features of the described embodiments may be combined in different ways, and many modifications and variations are possible within the scope of the appended claims. In the claims, any reference signs placed between parentheses shall exclude the presence of other elements or steps than those listed in the presence of a plurality of such elements.

## Claims

1. A corner fixating unit, comprising:
a first elongated half portion extending along a first central axis, and
a second elongated half portion extending along a second central axis,
wherein the first and second elongated half portions are pivotably fixed to each other at a respective inner end portion to allow pivotation of the first elongated half portion in relation to the second elongated half portion in a plane comprising both the first and second central axes,
wherein the first and second half portions each further comprises a tensioning depression at a respective outer portion extending along the respective first and second axis, the tensioning depression being adapted to engage a tensioning member.

2. The corner fixating unit according to claim 1, wherein each tensioning depression is at least partly tapered such that a depression depth increases from an outer portion of the depression in a direction towards said inner portion of each elongated half portion.

3. The corner fixating unit according to any of the preceding claims, wherein the first and second elongated half portions are pivotably coupled to each other via a pivotable pin joint.

4. A structural building, comprising:
the corner fixating member according to any of the preceding claims,
a first material profile comprising a first hollow structure adapted to receive the first elongated half portion of the corner fixating unit,
a second material profile comprising a second hollow structure adapted to receive the second elongated half portion of the corner fixating unit, and
a first and second tensioning member,
wherein said first and said second material profile each further comprises a tensioning member slot intersecting the respective first and second hollow structure so as to enable the first and second tensioning member to engage the first and second tensioning depression of the corner fixating unit respectively.

5. The structural building according to claim 5, wherein said first and second tensioning member slots are threaded and said first and second tensioning member are threaded so as to enable threaded engagement between the tensioning members and the tensioning member slots.

6. The structural building according to claim 6, wherein the tensioning member is a screw.

7. The structural building according to any of claims 4-6, wherein each tensioning member slot intersects the respective first and second hollow structure substantially perpendicularly.

8. The structural building according to any of claims 4-7, further comprising:
a corner stabilizing member,
wherein the corner stabilizing member is fixated to the first material profile and said second material profile further comprises a slot for receiving a portion of the corner stabilizing member fixated to the first material profile, or
wherein the first and second material profile each comprises a slot for receiving a respective portion of the corner stabilizing member.

9. The structural building according to any of claims 4-8, wherein in the engaged position the angle between the first and second central axis of the corner fixating member is within a range of 10-170 degrees, preferably within a range of 70-110 degrees.

10. The structural building according to any of claims 4-9, wherein in the material profiles are elongated structural columns.

11. The structural building according to claim 10, wherein the elongated columns are sized and adapted to be used for a structural building such as a pool roof or a conservatory structure.

12. The structural building according to any of claims 4-11, wherein the first and second material profiles are made of aluminum.

13. Use of the structural building according to any of claims 4-12 as a pool roof or a conservatory structure.

14. A method for assembling a building structure by fixating a first material profile to a second material profile, comprising the steps of:
providing a corner fixating unit according to any of claims 1-3;
providing first and second tensioning member;
inserting the first elongated half portion of the corner fixating unit into a first hollow structure of the first material profile;
inserting the second elongated half portion of the corner fixating unit into a second hollow structure of the second material profile;
inserting a first and second tensioning member into a respective tensioning member slot of each material profile, wherein each tensioning member slot intersecting the respective first and second hollow structure;
engaging the first and second tensioning depressions of the corner fixating unit with the first and second tensioning members respectively.

15. The method according to claim 14, wherein the tensioning depressions are tapered and the tensioning member slots comprise threads, further comprising the steps of:
rotating the first tensioning member in the first threaded tensioning member slot so as to engage the tapered tensioning depression of the first elongate half portion of the corner fixating unit, and
rotating the second tensioning member in the second threaded tensioning member slot so as to engage the tapered tensioning depression of the second elongate half portion of the corner fixating unit,
so that the first material profile is tightened to the second material profile by the engagement of the tensioning members and the tapered tensioning depressions.
